# EUROPEAN PATENT SPECIFICATION

(11) **EP 2 459 168 B1**
(45) Date of publication and mention of the grant of the patent: **09.09.2015**
(21) Application number: 10740394.1
(22) Date of filing: 20.07.2010
(51) Int. Cl.: A61K 9/00, A61K 49/00, A61K 9/50

(54) **METHOD FOR THE PREPARATION OF MICROPARTICLES WITH EFFICIENT BIOACTIVE MOLECULE INCORPORATION**
VERFAHREN ZUR HERSTELLUNG VON MIKROPARTIKELN MIT EFFIZIENTER AUFNAHME EINES BIOAKTIVEN MOLEKÜLS
PROCÉDÉ POUR LA PRÉPARATION DE MICROPARTICULES AVEC INCORPORATION EFFICACE DE MOLÉCULE BIOACTIVE

(30) Priority: 31.07.2009 EP 09166962
(43) Date of publication of application: 06.06.2012
(73) Proprietor: Koninklijke Philips N.V., 5656 AE Eindhoven (NL)
(72) Inventor: CHLON, Caecilia Hendrina Theodora, NL-5656 AE Eindhoven (NL); BOHMER, Marcel Rene, NL-5656 AE Eindhoven (NL)
(74) Representative: van Velzen, Maaike Mathilde
(86) International application number: PCT/IB2010/053307
(87) International publication number: WO 2011/013032

(56) References cited:
- WO-A1-2007/085990
- R. XU ET AL.: "Fabrication of indocyanine green encapsulated biodegradable microbubbles for structural and functional imaging of cancer", JOURNAL OF BIOMEDICAL OPTICS, vol. 14, no. 3, June 2009 (2009-06), pages 034020-1-034020-6, XP002661773,
- C. CHLON ET AL.: "Effect of molecular weight, crystallinity, and hydrophobicity on the acoustic activation of polymer-shelled ultrasound contrast agents", BIOMACROMOLECULES, vol. 10, April 2009 (2009-04), pages 1025-1031, XP002661774, cited in the application
- KOOIMAN K ET AL: "Oil-filled polymeric ultrasound contrast agent as local drug delivery system for lipophilic drugs", ULTRASONICS SYMPOSIUM, 2008. IUS 2008. IEEE, IEEE, PISCATAWAY, NJ, USA, 2 November 2008 (2008-11-02), pages 333-336, XP031443247, ISBN: 978-1-4244-2428-3
- J. CHAPPELL ET AL.: "Targeted delivery of nanoparticles bearing fibroblast growth factor-2 by ultrasonic microbubble destruction for therapeutic arteriogenesis", SMALL, vol. 4, no. 10, 2008, pages 1769-1777, XP002661775,
- HUALIN WANG ET AL.: "Hollow porous poly(lactic acid) microspheres", JOURNAL OF APPLIED POLYMER SCIENCE, vol. 107, 2007, pages 1189-1193, XP002661776,
- T. MAKUTA ET AL.: "Simple fabrication of hollow poly-lactic acid microspheres using uniform microbubbles as templates", MATERIAL LETTERS, vol. 63, 2009, pages 703-705, XP002661777,
- DALIA M. EL-SHERIF ET AL: 'Development of a novel method for synthesis of a polymeric ultrasound contrast agent' JOURNAL OF BIOMEDICAL MATERIALS RESEARCH vol. 66A, no. 2, 01 August 2003, pages 347 - 355, XP055098302 DOI: 10.1002/jbm.a.10586 ISSN: 0021-9304

## Description

### FIELD OF THE INVENTION

The invention relates to a method for the preparation of drug filled polymer microparticles comprising a gas core and shell, which particles are suitable as part of a therapeutic composition, especially for drug delivery.

### BACKGROUND OF THE INVENTION

Microparticle based ultrasound contrast agents are in use to enhance ultrasound contrast in medical imaging. Recent research demonstrates that they have therapeutic potential for drug delivery from the vasculature as well; microbubbles can increase the permeability of the endothelium and therefore lower the barrier for drug delivery from the vasculature. Drug delivery can also take place directly from drug loaded microbubbles themselves, which would allow a drastic change in the biodistribution with the potential to reduce side-effects of, for instance, cytotoxic agents.

USA-6,896,659 relates to a method of delivering a therapeutic agent to a localized region within a subject using ultrasound to trigger the release of the agent from hollow microbubbles having a specified set of mechanical properties. The agents disclosed in US-A-6,896,659 have a controlled fragility which is characterized by a uniform wall thickness to diameter ratio that defines discrete threshold power intensity. US-A-6,896,659 specifically discloses a single emulsion method for preparing the microbubbles wherein cyclooctane is used as a liquid forming the core in the creation of the microbubbles. This cyclooctane is in a later step removed by lyophilization.

The incorporation of drugs in polymeric spheres via a double emulsion methods is known from the prior art. (Sonsoles Diez et al, European Journal of Pharmaceutics and Biopharmaceutics 63 (2006) 188-197) (Diez et al.).

WO 2007/085990 discloses a method for producing a particle comprising a gas core and a shell and particles thus obtained.

Hualin Wang et al. (Journal of Applied Polymer Science, Vol. 107, 1189-1193 (2008) discloses hollow poly(lactic acid) microspheres and methods of producing the same.

Xu, R.X. et al. (Jounal of Biomedical Optics 14(3), May/June 2009) disclose the fabrication of indocyanine green encapsulated biodegradable microbubbles for structural and functional imaging of cancer.

El-Sherif, D. M. and Wheatley, M.A. (J. Biomed. Mater. Res. 66A: 347-355; 2003) disclose the development of a method for synthesis of a polymeric ultrasound contrast agent.

According to the double emulsion method described herein, polymeric spheres are synthesized by preparing a first emulsion by adding an aqueous drug containing solution into a polymer solution in an organic solvent. This first emulsion is subsequently emulsified again in an aqueous phase, after which the organic solvent is extracted.

### SUMMARY OF THE INVENTION

It is desirable to synthesize an agent with a single large gaseous core that can be acoustically activated at a pressure and frequency acceptable in ultrasound drug delivery, in combination with the capacity of this agent to comprise hydrophilic and/or hydrophopic drugs.

We have surprisingly found that the amount of incorporation of both hydrophobic and hydrophilic drugs is increased while obtaining a stable microparticle with a large hollow core by using specific ratios of polymer and solvents.

Therefore the invention in a first aspect relates to the following method for preparing biologically active agent filled polymer microparticles, said method comprising the steps of:
providing a first emulsion (A) by mixing an organic solvent (1), a biodegradable polyester, and an organic non-solvent for the polymer (2), wherein the weight ratio biodegradable polyester/organic non solvent is 1:10 to 1:1, and adding to this mixture from *up to 40% v*/*v* of an aqueous solution and wherein a drug is added to the organic mixture and or aqueous solution
preparing a second emulsion (B) by adding to this first emulsion (A) *excess* of an aqueous solution
applying conditions for volatizing the organic solvent (1),
isolating the microparticles from the aqueous solution,
applying conditions for removal of water,
applying conditions for removing of the non-solvent (2), wherein the non-solvent is selected from cyclooctane, cyclodecane, decane or a combination thereof.

By using this method, polymeric microparticles are obtained that combine high incorporation efficiency for hydrophilic and/or hydrophobic drugs with a large, preferably hollow, core. Microparticles formed via the method according to Diez et al lead to polymer spheres with a density higher than that of water, which thus can be centrifuged in the bottom of a vial. This implies that there is no large core present. This large core however, is essential for acoustic properties that can be used for the actual drug release via ultrasound. By performing the method according to the invention, small polymeric microparticles are obtained in a size range from 0.5-5 micrometers, more specifically from 1-3 micrometers that have a single gaseous core and are stable upon redispersion.

The drug is added in step a) to the organic solvent in the case of hydrophobic agents and in the aqeous phase for hydrophilic agents.

Also described are particles obtained by this method, their inclusion in contrast agents and therapeutic agents and to contrast agents or therapeutic compositions wherein the majority of particles can be activated by ultrasonic power that has an intensity in a range that is usual for ultrasound diagnostic imaging.

Particular and preferred aspects of the invention are set out in the accompanying independent and dependent claims. Features from the dependent claims may be combined with features of the independent claims and with features of other dependent claims as appropriate and not merely as explicitly set out in the claims.

### BRIEF DESCRIPTION OF THE DRAWINGS

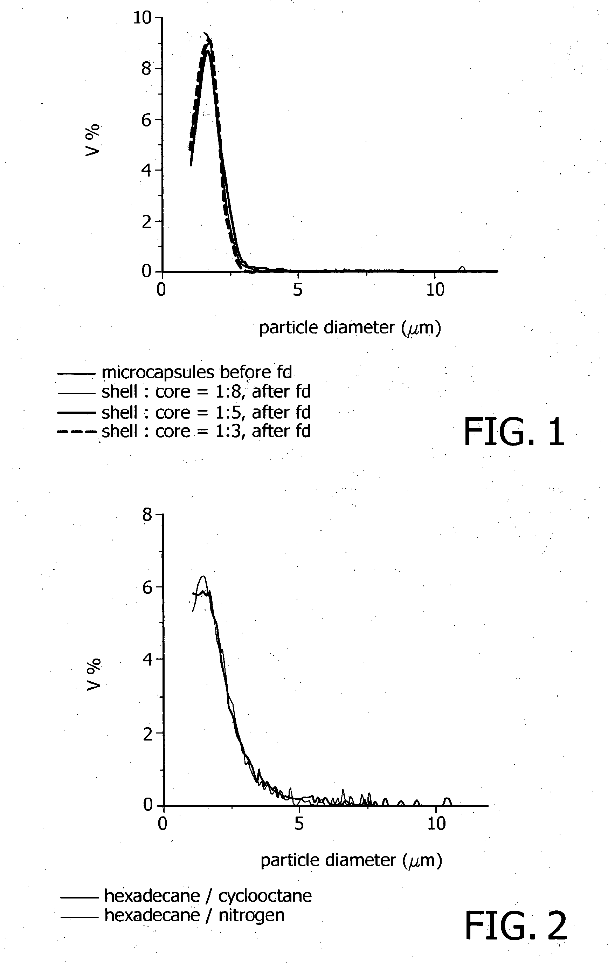

Fig 1-7: particle size distributions of microparticles obtained via the method according to the invention.

### DETAILED DESCRIPTION OF THE EMBODIMENTS

In the context of the invention the following definitions are used.

The present invention will be described with respect to particular embodiments and with reference to certain drawings but the invention is not limited thereto but only by the claims. The drawings described are schematic. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

### Method according to the invention

### Step a)

In the method according to the invention step (a) comprises providing a mixture comprising a shell forming polymer, a first solvent (1) and a second non- solvent (2).

This mixture is preferably made at a temperature between from 4 to 30°C, more preferred around room temperature.

In the context of the invention, the shell forming polymer is a biodegradable polyester, more preferably a biodegradable polyester selected from the group comprising polylactide either the L or the DL form, poly-lactide-co-glycolide and polycaprolacton, a combination thereof and block co-polymers thereof. Hollow polymer microparticles are obtained using biodegradable polyesters with a molecular weight range of 1.000 and 200.000 g/mol. More preferably, the molecular weight of the biodegradeble polyester is between 1500 and 20.000 and even more preferably between 1500 and 5000.

In a preferred embodiment, the biodegradable polyester comprises at least one moiety modified with at least one hydrophobic group that is preferably selected from the group comprising fluoride, alkyl chain comprising from 6 to 24 carbon atoms or a combination of these.

In the context of the invention solvent (1) is preferably a good solvent for the shell forming polymer. It is preferred that solvent (1) is a good solvent for the polymer forming the shell and non- solvent (2) is a bad solvent for the polymer forming the shell. Solvent (1) preferably dissolves in water to at least some extent. Solvent (1) is preferably relatively volatile.

Solvent (1) is preferably a solvent having a vapor pressure higher than water under the conditions of step (c), more preferably selected from the group comprising dichloromethane, dichloroethane or chloroform are examples of solvents that can be used, but also non-chlorinated solvents such as isopropylacetate can be used.

It is believed that non-solvent (2) is present to make particles comprising a gaseous core and a shell instead of solid particles. Therefore suitable compositions for solvent (2) are desirably relatively non volatile compositions wherein the chosen shell composition does not dissolve or only to a very low extent. Contrary to solvent 1 for non-solvent (2) it is highly preferred that the solubility in water is very low to zero.

Non-solvent (2) is selected from the group comprising organic compositions that have a vapor pressure significantly lower than water under the conditions of step (d). More preferred the vapor pressure of non-solvent (2) is at least 2 times lower, more preferably 4 times lower, than that of water under the conditions of step (d). The non-solvent (2) is selected such that its vapor pressure is still sufficiently high to enable removal under freeze-drying conditions optionally in combination with a suitable reduced-pressure that can easily be reached using well-known standard equipment.

This low vapor pressure and the low solubility will ensure that solvent (2) really stays inside the capsule being formed, leading in the end to form a capsule with a hollow gaseous space. Preferably the capsule comprises at least one hollow space. Most preferred the capsule comprises one main hollow space. If non-solvent (2) is disappearing from the capsule before the removal of solvent 1 is complete, the particles will show too much shrinking, thereby increasing their wall thickness, in step (c).

The non-solvent (2) is selected from the group comprising cyclooctane, cyclodecane, decane, or a combination thereof. In a most preferred embodiment, non-solvent (2) comprises cyclooctane, even more preferred the non-solvent (2) essentially consists of cyclooctane. In the context of the invention "essentially consists of" means that at least 80 wt%, preferably 90 to 100 wt% of the non-solvent (2) is cyclooctane.

Optionally in step (a) pre-mixtures are used of solvent (1) and (2) and of the shell composition and solvent (1).

Added to the mixture of organic solvent (1) and (2) and of the shell forming polymer is 0 to 0.4, more preferably approximately 0. 2 volumes of aqueous solution, resulting in emulsion A. Preferably, this aqueous solution is buffered.

To create an emulsion, preferably stirring or another form of agitation/shear forces is applied. Optionally further emulsification treatment is included to form an emulsion with the desired, preferably monodispersed, particle size distribution. Suitable equipment to obtain such emulsification treatment is for example selected from colloid mills, homogenizers, sonicaters.

Optionally the emulsion either before or after such treatments, is pressed through a glass filter. When desired such treatment may be repeated multiple times.

If apart from a gas phase a nonpolar liquid reservoir is desired in the microbubble, the organic solvent or non solvent can be mixed with an oil or alkane that cannot or with much more difficulty be freeze-dried out, for instance hexadecane.

Hydrophobic therapeutic compositions can be included in the core via this non-polar liquid reservoir. Hexadecane or paraffin oils may be used to solubilize a therapeutic composition in the core. Potential drugs that may be included in the particle core include anti-cancer drugs such as paclitaxel. We have surprisingly found that hexadecane is a very suitable carrier liquid for hydrophobic therapeutical compositions. We have found that such compositions easily stay dissolved or finely dispersed in hexadecane and these compositions will therefore incorporate inside the core of the particles in a remaining oil phase. Therefore the dissolved composition is released from the particles only after activation with ultrasound. Therefore in a preferred embodiment, the invention relates to the claimed particles further comprising at least one carrier liquid for a therapeutical composition. The most preferred carrier liquid is hexadecane.

Hydrophilic drugs are added to the first aqueous solution in emulsion A.

### Step b)

A further step (b) comprises combining the emulsion of step (a) with an aqueous composition, thereby forming an emulsion B of the mixture of step (a) in an aqueous phase.

Preferably the shell composition containing mixture of step (a) is added to an aqueous composition. To create an emulsion, preferably stirring or another form of agitation/shear forces is applied.

Optionally further emulsification treatment is included to form an emulsion with the desired, preferably monodispersed, particle size distribution.

Suitable equipment to obtain such emulsification treatment is for example selected from colloid mills, homogenizers, sonicaters.

Optionally the emulsion either before or after such treatments, is pressed through a glass filter. When desired such treatment may be repeated multiple times.

An alternative embodiment to create the desired particle size with a narrow distribution is using methods that produce monodisperse emulsions such as inkjet technology and emulsification using substrates with microchannels or micropores.

It is highly preferred that the conditions are controlled such that water and, especially, non-solvent (2) are not yet removed.

Optionally in step (a) or (b) a stabilizing composition is included. Such stabilizing composition is preferably selected from the group of surfactants and polymers comprising for example polyvinyl alcohol, albumin or a combination of at least two surfactants and/or polymers. If such stabilizing agent is included in the process, the process preferably includes a washing step after removal of solvent (1) to remove the stabilizer. The stabilizer is preferably used in a concentration between 0.1-20 %, more preferably between 5-15 %.

### Step c)

The conditions in step (c) are preferably such that the majority of non-solvent (2) is not yet removed, more preferred essentially no non-solvent (2) is removed. Hence it is preferred that in this step no measures are taken to reduce the pressure around the mixture such as by applying a vacuum.

A suitable way to remove solvent (1) is to increase the temperature for example to a temperature to a value a few degrees below the boiling point of the solvent to be removed-, or simply by stirring the mixture for a given amount of time.

Without wishing to be bound by any theory it is believed that whilst the solvent (1) vaporizes the concentration of the shell composition in the emulsion internal phase increases to over the solubility threshold and at such moment in time the shell composition will start to precipitate.

This precipitation then leads to the formation of a shell of polymer at the surface of the emulsion inner phase (emulsion droplet). It is believed that once the majority or all of solvent (1) has vaporized, a shell composition results which covers a core comprising non-solvent (2), water and optionally other ingredients that may have been added at an earlier stage of the process.

### Step d)

In this step, the microparticles are isolated from the aqueous phase and optionally washed to purify the particles. Separation of the particles can easily be facilitated by for example centrifugation, as the microparticles have a density that is lower than that of water.

### Step e)

In a further step (e) conditions are applied to remove water from the core. This is immediately followed by the removal of non-solvent (2) in step (f).

It is highly preferred that the removal of water and non-solvent (2) are separated in two different steps. In practice it may be unavoidable to have some overlap between these steps but overlap should preferably be avoided. Generally removal of water is obtained e.g. by freeze-drying techniques. Removal of non-solvent (2) may require further reduction of pressure.

The particles that result after step (e) are usually re-suspended in a suitable liquid before use. If the agent is to be used as a contrast agent or therapeutic agent for animals or humans, it is preferred that the particles are re-suspended in an aqueous physiological salt solution.

### Polymeric particle obtained by the method according to the invention

Disclosed is further a polymeric particle comprising a gas core and a polymeric shell wherein the particle has an average particle size of 0.5 to 5 micrometer. More preferably, at least 90% of the particles has a particle size of 0.5 to 5 micrometer, even more preferable more than 95 % of the particles has a particle size of 0.5 to 5 micrometer.

Such particles can be acoustically activated by application of ultrasound at a mechanical index of at most 3, more preferred at most 1.6, more preferred at most 1.2, even more preferred at most 1.0, even more preferred at most 0.8.

It is preferred that the activation sets off at a mechanical index above 0.2 , more preferred between 0.2 and 0.8, even more preferred at a lower limit of between 0.2 and 0.6.

For ultrasound mediated drug release applications it is desired that the polymeric microparticles are re- suspended in a suitable liquid forming a dispersion.

Most preferred the therapeutic composition comprises particles as described above wherein at least 80%, preferably 90 to 100% of the particles is acoustically activated upon application of ultrasound at a mechanical index, defined as the peak negative pressure divided by the square root of the frequency, of at most 3, more preferably below 2.

Generally this implies that at least 80% of the particles on application of ultrasound releases the gas and further ingredients from the core. It is highly desired that this release is taking place within a short time frame and within a small mechanical index range.

This acoustic activation can be monitored by the event count set up that is described in the examples. In this set up an activation event is qualified and counted when the amplitude of a received scattered signal (from an activated microparticle) is more than twice the noise level of the detection system.

In an exemplary embodiment, the invention relates to a therapeutic composition comprising particles comprising a gas core and polymeric shell, wherein at least 80% of the particles are activated by ultrasound energy, in a mechanical index window of 0.5 units, preferably a window of 0.4 units, more preferred 0.3 units within the mechanical index range of 0.01 to 3, more preferred 0.1 to 2, more preferred 0.4 to 2.

Preferably this activation is evidenced by an increase in the event count to at least 50 under the conditions specified in the examples.

This increase in event count preferably corresponds to an increase in echo intensity to at least 1000 times the initial value within the mechanical index window and range as described above.

A standard ultrasound transducer may be used to supply ultrasound energy. This sound energy may be pulsed but for maximal triggering of drug release it is preferred that the ultrasound energy is provided in a continuous wave. The gas containing particles can be imaged using several pulses of sound under clinically accepted diagnostic power levels for patient safety.

The invention is now illustrated by the following examples.

### Pharmaceutical composition

Microparticles disclosed herein are optionally formulated into diagnostic compositions, preferably for parenteral administration. For example, parenteral formulations advantageously contain a sterile aqueous solution or suspension of microparticles according to this invention. Various techniques for preparing suitable pharmaceutical solutions and suspensions are known in the art. Such solutions also may contain pharmaceutically acceptable buffers and, optionally, additives such as, but not limited to electrolytes (such as sodium chloride) or antioxidants. Parenteral compositions may be injected directly or mixed with one or more adjuvants customary in acoustic imaging.

Conventional excipients are pharmaceutically acceptable organic or inorganic carrier substances suitable for parenteral, enteral or topical application which do not deleteriously react with the agents. Suitable pharmaceutically acceptable adjuvants include but are not limited to water, salt solutions, alcohols, gum arabic, vegetable oils, polyethylene glycols, gelatine, lactose, amylose, magnesium stearate, talc, silicic acid, viscous paraffin, perfume oil, fatty acid monoglycerides and diglycerides, pentaerythritol fatty acid esters, hydroxy-methylcellulose, polyvinyl pyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, colouring, flavouring and/or aromatic substances and the like which do not deleteriously react with the active compounds.

For parenteral application, particularly suitable are injectable sterile solutions, preferably oil or aqueous solutions, as well as suspensions, emulsions, or implants, including suppositories. Ampoules are convenient unit dosages. The contrast agents containing microparticles are preferably used in parenteral application, e.g., as injectable solutions.

The therapeutic compositions are used in a conventional manner in ultrasound procedures. The diagnostic compositions are administered in a sufficient amount to provide adequate visualization and or drug delivery, to a warm-blooded animal either systemically or locally to an organ or tissues to be imaged, then the animal is subjected to the procedure. Such doses may vary widely, depending upon the diagnostic technique employed as well as the organ to be imaged.

### EXAMPLES

### Preparation of 100% gas filled microparticles with variations of shell thickness

0.1 g of pLLA (M_{w} 2400 g/mol) with a fluorinated end-group. prepared as described in Chlon et al. Biomacromolecules 2009 and cyclooctane (Aldrich C109401) in a ratio of 1:8, 1:5 or 1:3 were dissolved in 0.5 g dichloromethane. 120 µl of 30 mM TrisHCl buffer pH 7.5 was added and sonicated at room temperature two times 3 seconds (1 second interval) at 110 W. To this first emulsion 2 ml of 9 % polyvinyl alcohol (pVA, MW 13.000-23.000, Aldrich 363170) was added and homogenized using ultra thorax at 25.000 rpm at room temperature. The double emulsion was added dropwise to 8 ml 9 % pVA agitated using a magnetic stirrer at 660 rpm. After stirring for 3 hours at room temperature to remove the DCM the sample was centrifuged at 4000 rpm (G is about 1720 g) for 45 minutes. The top fraction was retrieved and washed for two more times for 20 minutes with milliQ water. The sample was rapidly frozen at -80 °C in a pre-cooled vial. Freeze-drying took place using a Christ epsilon 2-6 freeze-drier for 24 hours. After freeze-drying the system was filled with nitrogen. Samples were stored at 4 °C.

Before freeze-drying the pLLA-pFO microcaspules contain cyclooctane. After freeze-drying the nitrogen filled microparticles maintained their size distribution (Coulter counter) for all variations in shell thickness as shown in figure 1. Resuspending the freeze-dried microbubbles in an aqueous phase showed that they were all floating.

### 50% gas-filled pLLA-pFO microparticles

0.0166g of pLLA- (M_{w} 2400 g/mol) with a fluorinated end-group. prepared as described in Chlon et al. Biomacrmomolecules 2009 , 0.0417g of hexadecane (Aldrich H6703) and 0.0417g of cyclooctane (Aldrich C109401)were dissolved in 0.5 g dichloromethane. 120 µl of 30 mM TrisHCl buffer pH 7.5 was added and sonicated at room temperature two times 3 seconds (1 second interval) at 110 W. To this first emulsion 2 ml of 9 % polyvinyl alcohol (pVA, MW 13.000-23.000, Aldrich 363170) was added and homogenized using ultra thorax at 25.000 rpm at room temperature. The double emulsion was added dropwise to 8 ml 9 % pVA agitated using a magnetic stirrer at 660 rpm. After stirring for 3 hours at room temperature to remove the DCM the sample was centrifuged at 4000 rpm (G is about 1720 g) for 45 minutes. The top fraction was retrieved and washed for two more times for 20 minutes with milliQ water. The sample was rapidly frozen at -80 °C in a pre-cooled vial. Freeze-drying took place using a Christ epsilon 2-6 freeze-drier for 24 hours. After freeze-drying the system was filled with nitrogen. Samples were stored at 4 °C. The size distribution (Coulter counter) of the microparticles containing both hexadecane and cyclooctane was maintained after freeze-drying as shown in figure 2, where by means of lyophilization the cyclooctane was replaced by nitrogen, leading to half filled particles. Resuspending the freeze-dried microbubbles in an aqueous phase showed that they were all floating, indicating intact particles.

### 100% gas-filled pDLA-pFO microparticles

0.0166g of pDLA-pFO (M_{w} 4000 g/mol) and 0.0833g of cyclodecane (Fluka 28699) were dissolved in 0.5 g dichloromethane. 120 µl of 30 mM TrisHCl buffer pH 7.5 was added and sonicated at room temperature two times 3 seconds (1 second interval) at 110 W. To this first emulsion 2 ml of 9 % polyvinyl alcohol (pVA, MW 13.000-23.000, Aldrich 363170) was added and homogenized using ultra thorax at 25.000 rpm at room temperature. The double emulsion was added dropwise to 8 ml 9 % pVA agitated using a magnetic stirrer at 660 rpm. After stirring for 3 hours at room temperature to remove the DCM the sample was centrifuged at 4000 rpm (G is about 1720 g) for 45 minutes. The top fraction was retrieved and washed for two more times for 20 minutes with milliQ water. The sample was rapidly frozen at -80 °C in a pre-cooled vial. Freeze-drying took place using a Christ epsilon 2-6 freeze-drier for 24 hours. After freeze-drying the system was filled with nitrogen. Samples were stored at 4 °C.

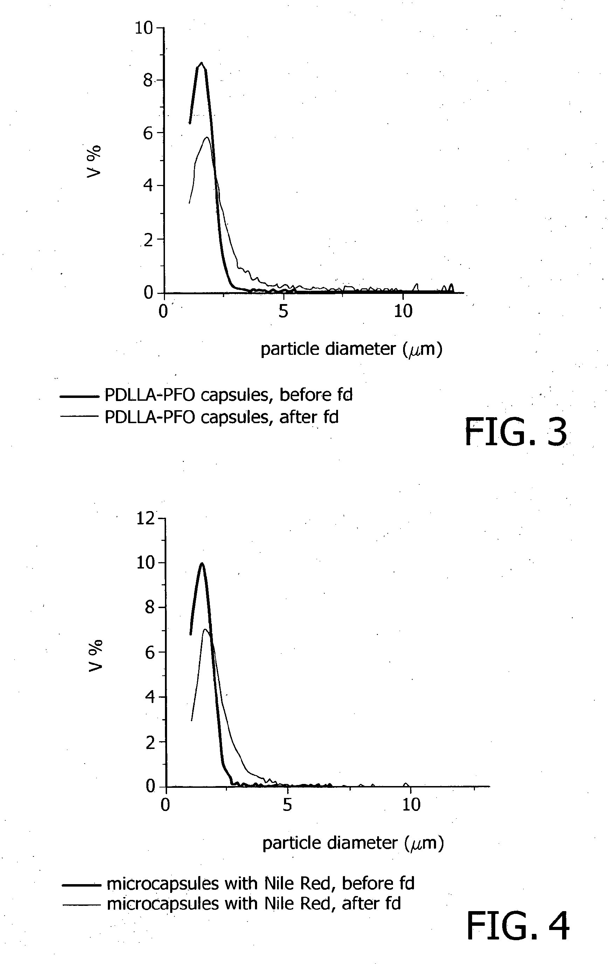

Microparticles made of amorphous pDLA-pFO showed after freeze-drying a size distribution (Coulter counter) comparable with its distribution before freeze-drying as shown in figure 3. Few aggregates were formed leading to a slight broadening of the size distribution peak. After resuspending these microbubbles in an aqueous phase the particles were all floating, indicating intact particles.

### Microbubbles filled with a model hydrophobic molecule

### 100% and 50% gas-filled microparticles loaded with Sudan Black

0.0166g pLLA-pFO (M_{w} 2400 g/mol) and 0.23mg of Sudan Black dissolved in 0.0833g alkane (either cyclooctane, Fluka 28699, or cyclooctane with hexadecane, Aldrich H6703, in a ratio 1:1) were dissolved in 0.5 g dichloromethane. 120 µl of milliQ water was added and sonicated at room temperature two times 3 seconds (1 second interval) at 110 W. To this first emulsion 2 ml of 9 % polyvinyl alcohol (pVA, MW 13.000-23.000, Aldrich 363170) was added and homogenized using ultra thorax at 25.000 rpm at room temperature. The double emulsion was added dropwise to 8 ml 9 % pVA agitated using a magnetic stirrer at 660 rpm. After stirring for 3 hours at room temperature to remove the DCM the sample was centrifuged at 4000 rpm (G is about 1720 g) for 45 minutes. The top fraction was retrieved and washed for two more times for 20 minutes with milliQ water. The sample was rapidly frozen at -80 °C in a pre-cooled vial. Freeze-drying took place using a Christ epsilon 2-6 freeze-drier for 24 hours. After freeze-drying the system was filled with nitrogen. Samples were stored at 4 °C.

The size distributions before and after freeze-drying of the microparticles containing 100% en 50% gas-filled particles with Sudan Black were comparable and below 5 micrometers. Resuspending the freeze-dried microbubbles in an aqueous phase showed that they were all floating, indicating intact particles. Sudan Black, as a hydrophobic model compound can successfully be incorporated in microparticles prepared with a double emulsion.

The encapsulation efficiency was determined by extracting the dye from the products in dodecane measuring the absorbance gave an incorporation efficiency of 84% for 100% gas filled microbubles and 93% for half-gas filled microbubbles. Samples made by the single emulsion method showed incorporation efficiencies of 46 and 76% for 100% gas-filled and 50% gas filled micorbubbles respectively (Kooiman et al, J. Contr. Rel. 2009)

### 100% gas-filled pLLA-pFO microparticles with the hydrophobic model compound Nile Red

0.0166g of pLLA-pFO (M_{w} 2400 g/mol) and 0.0833g of cyclooctane (Aldrich C109401)were dissolved in 0.5 g dichloromethane with dissolved Nile Red. 120 µl of 30 mM TrisHCl buffer pH 7.5 was added and sonicated at room temperature two times 3 seconds (1 second interval) at 110 W. To this first emulsion 2 ml of 9 % polyvinyl alcohol (pVA, MW 13.000-23.000, Aldrich 363170) was added and homogenized using ultra turrax at 25.000 rpm at room temperature. The double emulsion was added dropwise to 8 ml 9 % pVA agitated using a magnetic stirrer at 660 rpm. After stirring for 3 hours at room temperature to remove the DCM the sample was centrifuged at 4000 rpm (G is about 1720 g) for 45 minutes. The top fraction was retrieved and washed for two more times for 20 minutes with milliQ water. The sample was rapidly frozen at -80 °C in a pre-cooled vial. Freeze-drying took place using a Christ epsilon 2-6 freeze-drier for 24 hours. After freeze-drying the system was filled with nitrogen. Samples were stored at 4 °C.

The size distributions (Coulter counter) before and after freeze-drying of the microparticles containing Nile Red were comparable and shown in figure 4. Resuspending the freeze-dried microbubbles in an aqueous phase showed that they were all floating, indicating intact particles. Nile Red, as a hydrophobic model compound can successfully be incorporated in microparticles prepared with a double emulsion. Fluorescent microscopy shows incorporation of nile red in the shell

### 100% and 50% gas-filled pLLA-pFO microparticles loaded with taxol

0.0166g of pLLA-pFO (M_{w} 2400 g/mol)and 0.1g of alkane (either cyclooctane (Aldrich C109401), or cyclooctane with hexadecane, Aldrich H6703, in a ratio 1:1) were dissolved in 0.5 g 0.6% taxol solution in dichloromethane. 120 µl of 30 mM TrisHCl buffer pH 7.5 or pH 8.0 was added and sonicated at room temperature two times 3 seconds (1 second interval) at 110 W. To this first emulsion 2 ml of 9 % polyvinyl alcohol (pVA, MW 13.000-23.000, Aldrich 363170) was added and homogenized using ultra thorax at 25.000 rpm at room temperature. The double emulsion was added dropwise to 8 ml 9 % pVA agitated using a magnetic stirrer at 660 rpm. After stirring for 3 hours at room temperature to remove the DCM the sample was centrifuged at 4000 rpm (G is about 1720 g) for 45 minutes. The top fraction was retrieved and washed for two more times for 20 minutes with milliQ water. The sample was rapidly frozen at -80 °C in a pre-cooled vial. Freeze-drying took place using a Christ epsilon 2-6 freeze-drier for 24 hours. After freeze-drying the system was filled with nitrogen. Samples were stored at 4 °C.

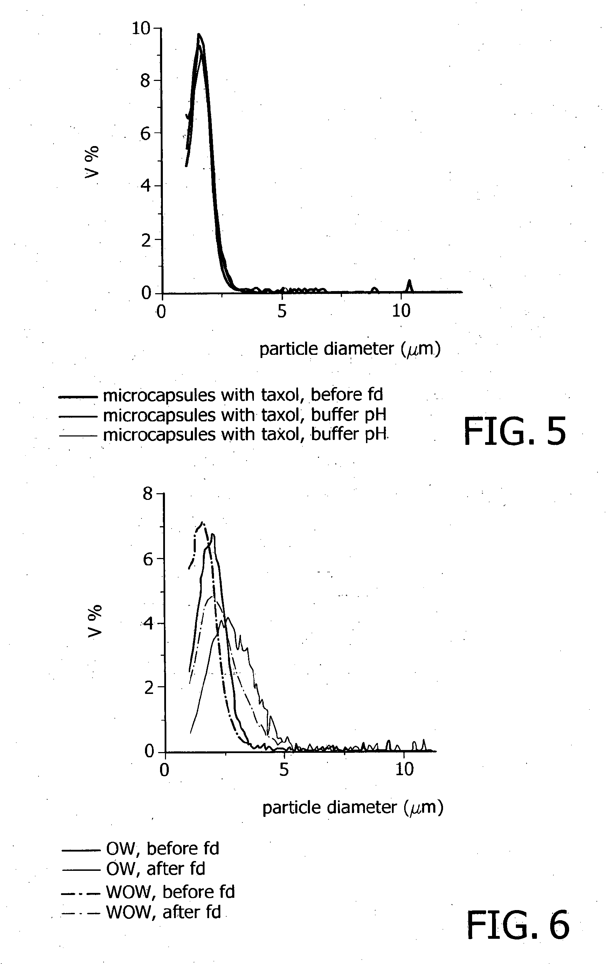

Figure 5 shows the size distributions (Coulter counter) for the 100% and 50% gas-filled particles made by the double emulsion technique both size distributions were in the range of 1-5 µm before freeze-drying. 50% gas-filled particles showed some aggregation which is well known for the particles with residual oil. The pH of the used buffer was not of any influence on the size distribution.

Particles made by a single emulsion technique showed a same trend in size distribution before and after freeze-drying, although particle made by the single emulsion method were in general slightly larger in size. This is shown in figure

After resuspending the 100% and 50% gas-filled particles, processed by either a single or double emulsion, in an aqueous phase, they all started to float, indicating intact particles.

Paclitaxel concentrations were determined by pevered phase liquid chromatography.

10 and 20 µL aliquots in dimethylformamide of all samples were separated using reversed phase liquid chromatography (RP-LC) on an Agilent 1200 HPLC system, consisting of a binary pump, a temperature-controlled well plate sampler and a diode array detector, equipped with a Phenyl-hexyl (4.6*100mm, 3.5µm particles) column applying a 20 minute linear gradient of B (0.1% FA in ACN) in A (0.1% FA in water) at a flow rate of 0.7 mL/min.

Eluting compounds were subsequently analysed using UV detection at 254 nm and an Agilent ESI-ion trap (MSD) mass spectrometer capable of performing tandem mass spectrometry measuring in the alternating (switching between positive and negative) mode in the mass range *m*/*z* 200-2000.

The resulting encapsulation efficiencies were given in Table I, as a reference the incorporation efficiency in single emulsion microparticles, as described in Kooiman et al. J. Controled Release 2009, is given.

**Table 1: Taxol loading efficiency for microparticles prepared by single or double emulsion**

| pLLA-pFO particles | Taxol loading efficiency |
|---|---|
| *Single emulsion* | |
| 50% gas-filled | 15% |
| *Double emulsion* | |
| 100% gas-filled, buffer pH 7.5 | 21% |
| 100% gas-filled, buffer pH 8.0 | 21% |
| 50% gas-filled, buffer pH 7.5 | 39% |
| 50% gas-filled, buffer pH 8.0 | 58% |

Microparticles prepared with a double emulsion technique showed much higher paclitaxel loading efficiencies than for particles prepared with the single emulsion method. As discussed before regarding the double emulsion, the taxol crystallized not only in the (outer) aqueous phase during particle formation, but crystallization also took place to a significant extent on the surface of the encapsulated water, leading to a more efficient taxol encapsulation. The taxol loading efficiency for 50% gas-filled particles increased from 15% to 39% when prepared by a double emulsion. Increasing the pH of the buffered solution to 8.0 increased the loading efficiency even further to 58%.

For contrast agents with drug delivery from the vasculature it is preferred to inject microbubbles consisting of no additional alkane, like hexadecane. Although hexadecane is not able to keep the taxol dissolved in the capsule, introduction of this oil besides cyclooctane significantly increased the taxol encapsulation efficiency. Even when no hexadecane is incorporated the 100% gas-filled particles still showed better encapsulation results than for the 50% gas-filled microbubbles prepared with a single emulsion.

### 100% gas-filled pLLA-pFO microparticles with the hydrophilic model compound dextran FITC

0.0166g of pLLA-pFO(M_{w} 2400 g/mol) and 0.0833g of cyclooctane (Fluka 28699) were dissolved in 0.5 g dichloromethane. 120 µl of 4mg/ml Dextran-FITC pH 4.0 was added and sonicated at room temperature two times 3 seconds (1 second interval) at 110 W. To this first emulsion 2 ml of 9 % polyvinyl alcohol (pVA, MW 13.000-23.000, Aldrich 363170) pH 4.0 was added and homogenized using ultra thorax at 25.000 rpm at room temperature. The double emulsion was added dropwise to 8 ml 9 % pVA at pH4.0 agitated using a magnetic stirrer at 660 rpm. After stirring for 3 hours at room temperature to remove the DCM the sample was centrifuged at 4000 rpm (G is about 1720 g) for 45 minutes. The top fraction was retrieved and washed for two more times for 20 minutes with milliQ water. The sample was rapidly frozen at -80 °C in a pre-cooled vial. Freeze-drying took place using a Christ epsilon 2-6 freeze-drier for 24 hours. After freeze-drying the system was filled with nitrogen. Samples were stored at 4 °C.

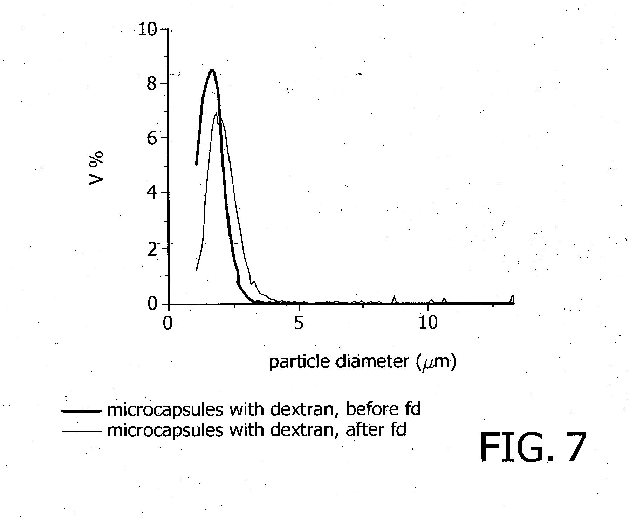

The size distributions (Coulter counter) before and after freeze-drying of the microparticles containing dextran FITC were comparable and shown in figure 7. Resuspending the freeze-dried microbubbles in an aqueous phase showed that they were all floating, indicating intact particles. Dextran, as a hydrophilic model compound can successfully be incorporated in microparticles prepared with a double emulsion.

By measuring the fluorescence in the supernatant the incorporation efficiency was established to be 43%. Fluorescence microscopy demonstrates the presence in the shell.

## Claims

1. A method for preparing drug filled polymer microparticles, said method comprising the steps of:
a) providing a first emulsion (A) by mixing an organic solvent (1), a biodegradable polyester, and an organic non-solvent for the polyester polymer (2), wherein the weight ratio biodegradable polyester/organic non solvent is 1:10 to 1:1, and
adding to this mixture up to 40% v/v of an aqueous solution and wherein a drug is added to the organic mixture and/or aqueous solution,
b) preparing a second emulsion (B) by adding to this first emulsion (A) excess of an aqueous solution,
c) volatizing the organic solvent (1),
d) isolating the microparticles from the aqueous solution,
e) removing water,
f) removing the non-solvent (2),
wherein the non-solvent is selected from cyclooctane, cyclodecane, decane, or a combination thereof.

2. The method according to claim 1, wherein drug filled polymer microparticles have an average microparticle size between 0.5 and 5 µm.

3. A method according to claim 1, wherein the biodegradable polyester has a molecular weight between 1.000 and 200.000 g/mol.

4. The method according to claim 1, wherein the drug is hydrophilic.

5. The method according to claim 1, wherein the drug is hydrophobic.

6. The method according to claim 1, wherein a hydrophobic drug is added to the organic mixture and a hydrophilic drug is added to the aqueous solution.

7. The method according to claim 1, wherein the ratio biodegradable polyester/organic non solvent (2) is 1:8 to 1:3.

8. The method according to any of the preceding claims, wherein a non solvent (3) that is not removed in step e) is added to step a).

9. The method according to any of the previous claims wherein the polymer is selected from the group comprising polylactide either in the L or DL form, poly-lactide-co-glycolide, polycaprolacton, a combination thereof, or a block co-polymer thereof.

10. Method according to claim 9, wherein the polymer comprises at least one moiety modified with at least one hydrophobic group that is preferably selected from the group comprising fluoride, alkyl chain comprising from 6 to 24 carbon atoms or a combination of these.

## Patentansprüche

1. Verfahren zur Herstellung von mit Arzneimittel gefüllten Polymermikropartikeln, wobei das Verfahren die folgenden Schritte umfasst, wonach::
a) eine erste Emulsion (A) durch Mischen eines organischen Lösungsmittels (1), eines biologisch abbaubaren Polyesters und eines organischen Nichtlösungsmittels (2) für das Polyesterpolymer vorgesehen wird, wobei das Gewichtsverhältnis zwischen biologisch abbaubarem Polyester und organischem Nichtlösungsmittel 1:10 bis 1:1 beträgt, und
zu diesem Gemisch bis zu 40% v/v einer wässrigen Lösung beigefügt wird, und wobei zu dem organischen Gemisch und/oder der wässrigen Lösung ein Arzneimittel zugegeben wird,
b) eine zweite Emulsion (B) durch Zugeben eines Überschusses einer wässrigen Lösung zu dieser ersten Emulsion (A) hergestellt wird,
c) das organische Lösungsmittel (1) verflüchtigt wird,
d) die Mikropartikel von der wässrigen Lösung isoliert werden,
e) Wasser entfernt wird,
f) das Nichtlösungsmittel (2) entfernt wird,
wobei das Nichtlösungsmittel aus Cyclooctan, Cyclodecan, Decan oder einer Kombination daraus ausgewählt wird.

2. Verfahren nach Anspruch 1, wobei mit Arzneimittel gefüllte Polymermikropartikel eine durchschnittliche Mikropartikelgröße zwischen 0,5 und 5 µm aufweisen.

3. Verfahren nach Anspruch 1, wobei der biologisch abbaubare Polyester ein Molekulargewicht zwischen 1,000 und 200,000 g/mol aufweist.

4. Verfahren nach Anspruch 1, wobei das Arzneimittel hydrophil ist.

5. Verfahren nach Anspruch 1, wobei das Arzneimittel hydrophob ist.

6. Verfahren nach Anspruch 1, wobei ein hydrophobes Arzneimittel zu dem organischen Gemisch zugegeben wird und ein hydrophiles Arzneimittel zu der wässrigen Lösung zugegeben wird.

7. Verfahren nach Anspruch 1, wobei das Verhältnis zwischen biologisch abbaubarem Polyester und organischem Nichtlösungsmittel (2) 1:8 bis 1:3 beträgt.

8. Verfahren nach einem der vorangegangenen Ansprüche, wobei ein Nichtlösungsmittel (3), das in Schritt e) nicht entfernt wird, zu Schritt a) zugegeben wird.

9. Verfahren nach einem der vorangegangenen Ansprüche, wobei das Polymer aus der Gruppe, umfassend Polylactid in entweder der L- oder DL-Form, Poly-Lactid-Coglycolid, Polycaprolacton, eine Kombination daraus, oder ein Block-Copolymer daraus, ausgewählt wird.

10. Verfahren nach Anspruch 9, wobei das Polymer mindestens eine Komponente umfasst, die mit mindestens einer hydrophoben Gruppe modifiziert wird, die vorzugsweise aus der Gruppe, umfassend Fluorid, Alkylkette mit 6 bis 24 Kohlenstoffatomen oder eine Kombination aus diesen, ausgewählt wird.

## Revendications

1. Procédé pour préparer des microparticules polymères remplies de médicament, ledit procédé comprenant les étapes de :
a) la fourniture d'une première émulsion (A) en mélangeant un solvant organique (1), un polyester biodégradable, et un solvant non organique pour le polymère polyester (2), dans lequel le rapport pondéral de polyester biodégradable/solvant non organique est 1:10 à 1:1, et
ajouter à ce mélange jusqu'à 40 % v/v d'une solution aqueuse et dans lequel un médicament est ajouté au mélange organique et/ou à la solution aqueuse,
b) la préparation d'une seconde émulsion (B) en ajoutant à cette première émulsion (A) un excès d'une solution aqueuse,
c) la volatilisation du solvant organique (1),
d) l'isolation des microparticules par rapport à la solution aqueuse,
e) l'élimination d'eau,
f) l'élimination du non-solvant (2),
dans lequel le non-solvant est sélectionné parmi du cyclooctane, du cyclodécane, du décane, ou une association de ceux-ci.

2. Procédé selon la revendication 1, dans lequel des particules polymères remplies de médicament possèdent une taille moyenne des microparticules entre 0,5 et 5 µm.

3. Procédé selon la revendication 1, dans lequel le polyester biodégradable présente un poids moléculaire entre 1,000 et 200,000 g/mol.

4. Procédé selon la revendication 1, dans lequel le médicament est hydrophile.

5. Procédé selon la revendication 1, dans lequel le médicament est hydrophobe.

6. Procédé selon la revendication 1, dans lequel un médicament hydrophobe est ajouté au mélange organique et un médicament hydrophile est ajouté à la solution aqueuse.

7. Procédé selon la revendication 1, dans lequel le rapport de polyester biodégradable/solvant non organique (2) est 1:8 à 1:3.

8. Procédé selon l'une quelconque des revendications précédentes, dans lequel un non solvant (3) qui n'est pas éliminé dans l'étape e) est ajouté à l'étape a).

9. Procédé selon l'une quelconque des revendications précédentes dans lequel le polymère est sélectionné parmi le groupe comprenant un polylactide dans la forme L ou DL, un poly-lactide-co-glycolide, un polycaprolactone, une association de ceux-ci, ou un co-polymère à blocs de ceux-ci.

10. Procédé selon la revendication 9, dans lequel le polymère comprend au moins un groupe caractéristique modifié avec au moins un groupe hydrophobe qui est de préférence sélectionné parmi le groupe comprenant du fluor, une chaîne alkyle comprenant de 6 à 24 atomes de carbone ou une association de ceux-ci.
